# FASCICULE DE BREVET EUROPEEN

(11) **EP 1 290 983 B1**
(45) Date de publication et mention de la délivrance du brevet: **08.04.2009**
(21) Numéro de dépôt: 02358022.8
(22) Date de dépôt: 09.09.2002
(51) Int. Cl.: A61B 19/02, A61L 9/00

(54) **Armoire de stockage hyperaseptique, en particulier pour endoscopes**
Schrank zum hyperaseptischen Lagern, insbesonders für Endoskope
Cabinet for hyperaseptically storing, in particular for endoscopes

(30) Priorité: 10.09.2001 FR 0111690
(43) Date de publication de la demande: 12.03.2003
(73) Titulaire: Hysis Medical S.A., 13400 Aubagne (FR)
(72) Inventeur: Beyly, Jean-Richard, 13011 Marseille (FR); Lafoucriere, Didier, 13008 Marseille (FR)
(74) Mandataire: Marek, Pierre

(56) Documents cités:
- EP-A- 0 556 907
- WO-A-01/36068
- DE-A- 1 541 352
- DE-U- 8 615 367
- US-A- 4 202 676

## Description

La présente invention concerne une armoire de stockage hyperaseptique, en particulier pour endoscopes, afin de les maintenir, jusqu'à leur prochaine réutilisation, au niveau d'aseptie obtenu après la phase de décontamination/séchage du protocole de désinfection appliqué à ce type de matériels, conformément aux règlements en vigueur.

Toutefois, l'application avantageuse susmentionnée n'est pas limitative, l'armoire de stockage hyperaseptique selon l'invention pouvant être utilisée pour maintenir différents articles au niveau de désinfection imposé par les normes et règlements, tels que d'autres articles de l'instrumentation chirurgicale, etc.

Réglementairement, les endoscopes doivent être stockés dans un endroit propre et sec, à l'abri de toute source de contamination microbienne. Toutefois, la pratique quotidienne observée dans les établissements hospitaliers, permet de constater que cette recommandation n'est pratiquement jamais respectée, faute de matériel adapté.

On connaît (FR-2.710.350), une armoire pour le séchage et l'aseptisation de vêtements, comportant :
- une paroi verticale parallèle au fond de l'armoire et délimitant avec celui-ci un couloir vertical ;
- un ensemble technique disposé en partie haute, et comprenant un condenseur obturant la face supérieure de l'armoire, en dehors du couloir, une lampe à ozone montée au-dessus du condenseur, et un ventilateur de circulation d'air aspirant l'air dans l'armoire à travers le condenseur et le refoulant dans le couloir après passage sur une résistance chauffante,
- des orifices de communication ménagés dans la paroi verticale séparant le volume utile de l'armoire du couloir.

Cette armoire établit donc une circulation d'air en circuit fermé, l'air étant soufflé de haut en bas dans le couloir disposé à la partie postérieure de l'armoire, pénétrant dans celle-ci par les orifices ménagés dans la paroi verticale et étant repris en partie haute de ladite armoire où se trouve l'ensemble technique de séchage et d'aseptisation.

Le niveau d'aseptisation qu'il est possible d'obtenir avec un matériel de ce genre est nettement insuffisant pour qu'il soit envisageable de le retenir pour le maintien d'une atmosphère bactériologiquement propre dans une armoire de stockage d'endoscopes préalablement nettoyés et désinfectés.

En effet, pour ce type d'application, l'action bactéricide et virucide de l'ozone est limitée, tandis que l'injection d'air dans le compartiment de stockage, à travers une paroi verticale de l'armoire, et le sens de circulation d'air en circuit fermé, génère, à l'intérieur de ladite armoire, des courants turbulents ascendants qui ne passent pas dans la partie inférieure de celle-ci, de sorte qu'il subsiste, dans la partie basse du compartiment de stockage, un important volume d'air qui n'est pas ou peu concerné par l'action de l'ensemble technique de séchage et d'aseptisation. D'autre part, l'écoulement turbulent de l'air, favorise la propagation des particules contaminées.

Dans le document US - 4 202 676, est décrite une armoire mobile aseptisée dont le but est de permettre aux personnes qui procèdent à des recherches ou tests sur les animaux ou sur les cultures de tissus ou de végétaux, de travailler dans un milieu aseptisé, tout en protégeant les sujets de ces recherches ou tests, de toute contamination.

Cette armoire mobile comprend une chambre aseptisée ou chambre blanche et un compartiment technique renfermant les composants techniques permettant la production d'air bactériologiquement propre et la mise en circulation de cet air aseptisé dans ladite chambre blanche.

Un tel dispositif peut correspondre à la fonction spécifique pour laquelle il a été conçu et proposé, mais il est insuffisant pour garantir le maintien à l'état stérile d'articles spéciaux tels que des endoscopes stockés après le protocole réglementaire de stérilisation auquel sont soumis ces appareils.

Par conséquent, le dispositif décrit dans le document US - 4 202 676 n'est pas approprié pour le stockage des endoscopes préalablement stérilisés et en attente d'une prochaine réutilisation.

La présente invention a notamment pour objet de remédier aux inconvénients ou insuffisances des armoires de stockage aseptiques proposées à ce jour et, plus particulièrement, de mettre à disposition des services hospitaliers (hôpitaux, cliniques, etc.), une armoire de stockage hyperaseptique pour les endoscopes, après la phase de décontamination séchage du protocole de désinfection appliqué à ce type de matériel, conformément au règlement en vigueur.

L'invention met en oeuvre une armoire de stockage aseptique du genre comprenant un compartiment technique contenant les composants techniques nécessaires à la production d'air bactériologiquement propre, une enceinte à atmosphère aseptisée pour la réception et le stockage des articles à préserver d'une contamination, et des moyens permettant d'établir une circulation en boucle de l'air à travers ledit compartiment et ladite enceinte, cette armoire étant agencée de sorte à ce que l'air traité soit introduit à la partie supérieure de l'enceinte de stockage et poussé à circuler de haut en bas, sous forme de flux laminaires et en surpression à travers ladite enceinte de stockage jusqu'à la base de cette dernière d'où il repasse dans la partie inférieure du compartiment technique dans lequel il circule de bas en haut, à travers lesdits composants techniques, ladite armoire comprenant encore des manches verticales stériles, à usage unique, disposées verticalement dans l'enceinte de rangement, ces manches stériles (aussi appelées "saches") étant constituées par des gaines de forme allongée ouvertes à leurs extrémités supérieure et inférieure, et dimensionnées pour autoriser le logement d'au moins la partie invasive d'un endoscope désinfecté, l'extrémité supérieure de ces saches étant munie d'un moyen permettant leur suspension à un support installé en partie haute de ladite enceinte de rangement, lesdites saches permettant ainsi de canaliser l'écoulement laminaire du flux d'air aseptisé autour des endoscopes logés dans ces dernières.

L'armoire hyperaseptique selon l'invention permet de stocker, en station verticale et unitairement, des endoscopes désinfectés, et de garantir ainsi qu'il ne peut y avoir une contamination croisée de chacun d'eux avec d'éventuels autres endoscopes ou appareils ou parties d'appareils suspendus dans ladite armoire. Elle constitue, de la sorte, un dispositif de prévention efficace de la recontamination d'un endoscope médical lors de son stockage postérieurement à sa désinfection par un procédé dédié et adapté. D'autre part, les manches sont aptes à canaliser un flux d'air aseptisé qui sera de préférence contrôlé d'un point de vue hygiène de façon à mettre l'intérieur de chaque manche, et donc l'environnement proche de l'endoscope, en situation isolée de l'environnement extérieur pouvant être contaminant.

Selon un mode d'exécution avantageux, un registre correctement calibré installé en partie basse du compartiment technique, permet une admission d'air neuf dans ledit compartiment, lequel se mélange ainsi, en partie basse de ce dernier, à l'air recyclé provenant de l'enceinte de stockage, cet apport d'air neuf permettant la mise en surpression de l'enceinte de stockage et la dissipation d'une partie de la chaleur induite par le fonctionnement de certains dispositifs logés dans le compartiment technique.

Selon un autre mode de réalisation, l'aérobiodécontamination du compartiment de stockage est obtenue au moyen d'une chaîne de traitement mettant en oeuvre un procédé physico-chimique et un procédé mécanique, comprenant :
- une pré-filtration sur filtre moyenne efficacité, par exemple de type gravimétrique G3, selon EN 779 (synthétique plissé) ;
- une filtration sur filtre haute efficacité, par exemple de type gravimétrique G4 selon EN 779 (synthétique plan) ;
- une irradiation dans une chambre équipée d'une rampe bactériologique UV
- une filtration terminale sur filtre très haute efficacité 99,99 à 99,999 DOP pour des particules à 0,3µ, par exemple sur filtre de type H14 selon EN 1822 (média fibre de verre Mini-pli).

Selon un autre mode d'exécution, l'enceinte ou compartiment de stockage est équipée, en partie haute, d'un répartiteur apte à être connecté, d'une part, à une source d'air médical fourni par l'établissement hospitalier et, d'autre part, à la sonde tubulaire des endoscopes. L'air médical canalisé, via ledit répartiteur, vers les endoscopes au moyen d'un régulateur de pression et d'une électrovanne, sert à parfaire le séchage desdits endoscopes.

Grâce aux dispositions caractéristiques ci-dessus, les endoscopes stockés dans l'armoire stérile, sont effectivement maintenus au niveau d'aseptie obtenu après la phase de décontamination/séchage du protocole de désinfection appliqué à ce type d'instruments, jusqu'à leur prochaine réutilisation.

Les buts, caractéristiques et avantages ci-dessus, et d'autres encore, ressortiront mieux de la description qui suit et des dessins annexés dans lesquels :
La figure 1 est une représentation synoptique du procédé de traitement de l'air mis en oeuvre dans l'armoire de stockage hyperaseptique.
La figure 2 est une vue de face montrant l'agencement intérieur d'une armoire de stockage, selon un premier mode de mise en oeuvre de l'invention.
La figure 3 est une vue de côté montrant l'équipement intérieur du module ou compartiment de traitement de l'air de cette armoire.
La figure 4 est une vue de côté montrant l'arrangement intérieur du module ou enceinte de rangement ou stockage de l'armoire.
La figure 5 est une vue en perspective illustrant plus spécialement un exemple avantageux de réalisation d'un dispositif de suspension des endoscopes et de distribution d'air médical.
La figure 6 est une vue en plan de la grille d'évacuation de l'air soufflé.
La figure 7 est une vue en plan du dispositif de direction de l'air soufflé.
La figure 8 est une vue en élévation montrant un endoscope positionné dans une manche stérile, selon l'invention.
La figure 9 est une vue de détail en perspective, illustrant le système d'accrochage équipant l'extrémité supérieure des manches stériles.
La figure 10 est une vue en perspective montrant un mode d'exécution d'une manche stérile équipée de ce système d'accrochage.
La figure 11 est une vue en perspective illustrant un exemple de configuration d'armoire de stockage hyperaseptique selon l'invention.
La figure 12 est une vue en perspective montrant un exemple d'agencement intérieur de cette armoire.
La figure 13 est une vue de face montrant l'agencement intérieur d'une armoire de stockage, selon un deuxième mode de réalisation de l'invention.
La figure 14 est une vue en perspective et à plus petite échelle de cette armoire dont un tiroir ou rack de suspension est montré en position ouverte.
La figure 15 est une vue de côté montrant l'arrangement intérieur d'un compartiment de rangement de l'armoire.

On se réfère auxdits dessins pour décrire des exemples intéressants, bien que nullement limitatifs, de réalisation de l'armoire de stockage hyperaseptique selon l'invention.

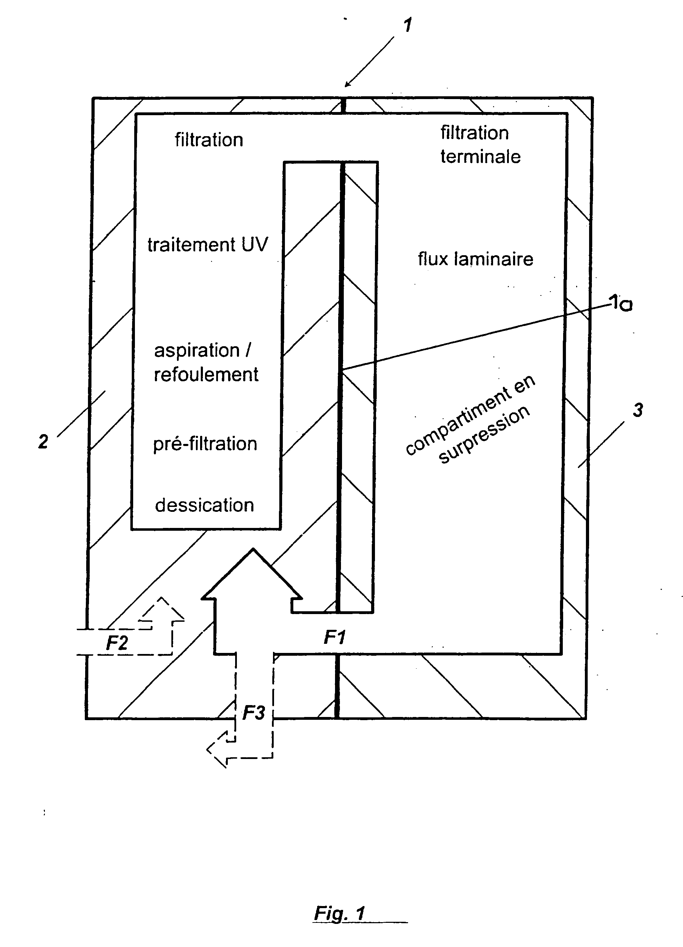

Selon le procédé mis en oeuvre dans l'armoire de stockage hyperaseptique de l'invention, on produit une circulation en boucle d'air aseptisé, selon flèche F1 (figure 1), dans une armoire de stockage aseptique 1, comprenant un compartiment technique 2 de traitement de l'air et une enceinte de rangement 3 destinée à recevoir les articles à maintenir au niveau d'aseptie souhaité, circulation suivant laquelle l'air aseptisé refoulé par le compartiment technique est introduit dans l'enceinte de rangement, à la partie supérieure de celle-ci, et poussé à circuler de haut en bas, sous forme de flux laminaire et en état de surpression à travers ladite enceinte de rangement 3, jusqu'à la partie basse de cette dernière d'où il est réintroduit dans la partie inférieure dudit compartiment technique 2.

Selon un mode de mise en oeuvre préféré, on introduit, suivant flèche F2, de l'air extérieur dans la partie inférieure du compartiment technique 2, lequel se mélange ainsi, en partie basse de ce dernier, à l'air recyclé provenant de l'enceinte de rangement 3, cet apport d'air extérieur permettant de créer une surpression dans cette dernière et d'absorber une partie de la chaleur générée par le fonctionnement de certains des dispositifs installés dans le compartiment technique et décrits ci-après, l'excèdent d'air étant évacué selon flèche F3.

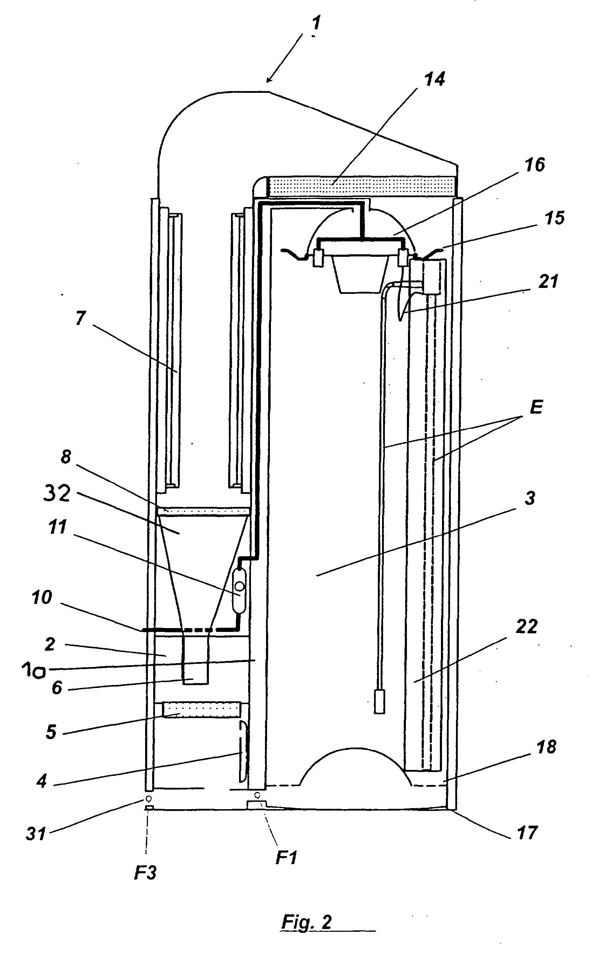

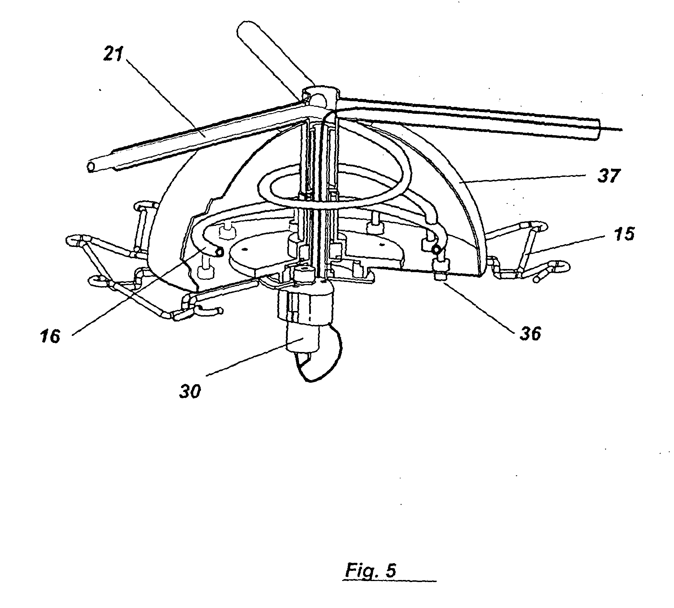

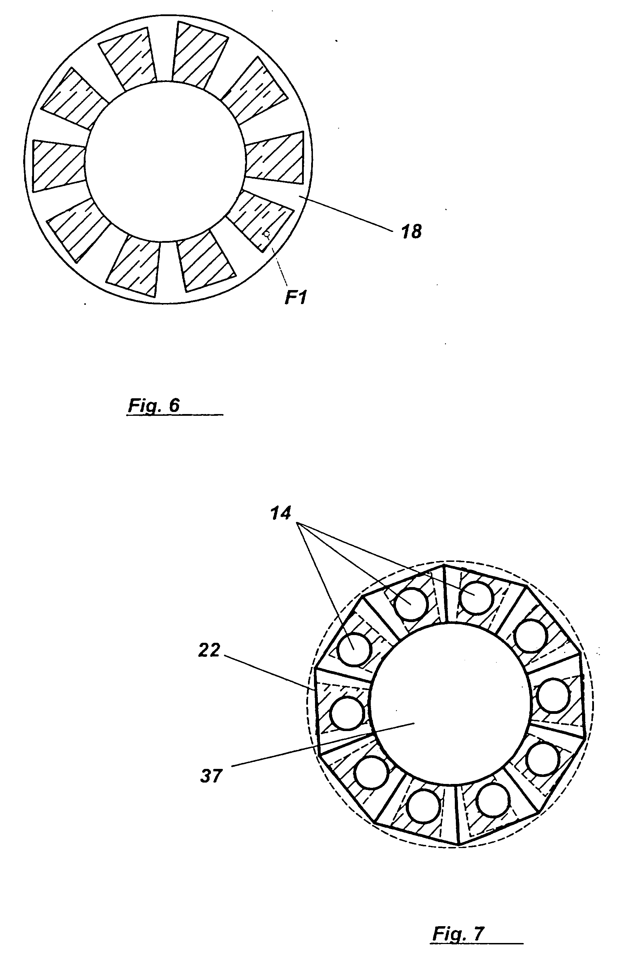

Selon le mode d'exécution illustré aux figures 2 à 7 et 12, l'armoire de stockage 1 comprend :
- un compartiment technique 2 à ouverture frontale contenant les organes techniques nécessaires aux conditions de fonctionnement de ladite armoire ; et
- une enceinte de rangement 3 à atmosphère contrôlée pour le stockage des endoscopes E ou autres articles, ce compartiment étant constitué d'un ou plusieurs volumes facilement accessibles et nettoyables.

Le compartiment technique 2 et l'enceinte de rangement 3 sont séparés par une cloison 1a, de préférence verticale.

Le compartiment technique 2 renferme, de bas en haut :
- un dispositif de déshydratation 4 qui peut être constitué par un dessiccateur chimique, ou par une batterie froide de déshumidification ;
- un filtre de pré-filtration 5, par exemple de type G3, selon EN 779 ;
- un dispositif d'aspiration/refoulement 6, par exemple constitué par un moto-ventilateur électrique ;
- un divergeant 32 ;
- un filtre 8, par exemple de type G4 selon EN 779, pour la filtration de l'air soufflé ;
- une batterie de tubes UV 7 pour le traitement de l'air ;

Le divergeant 32 positionné au-dessous de la batterie de tubes UV assure une répartition homogène du flux d'air traversant le filtre 8, sur ces derniers.

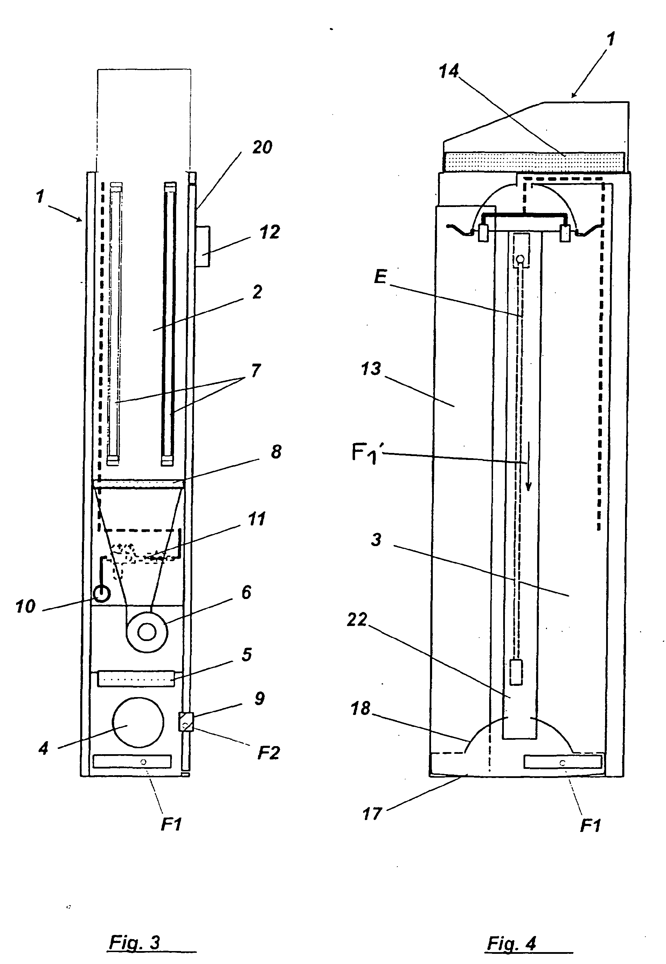

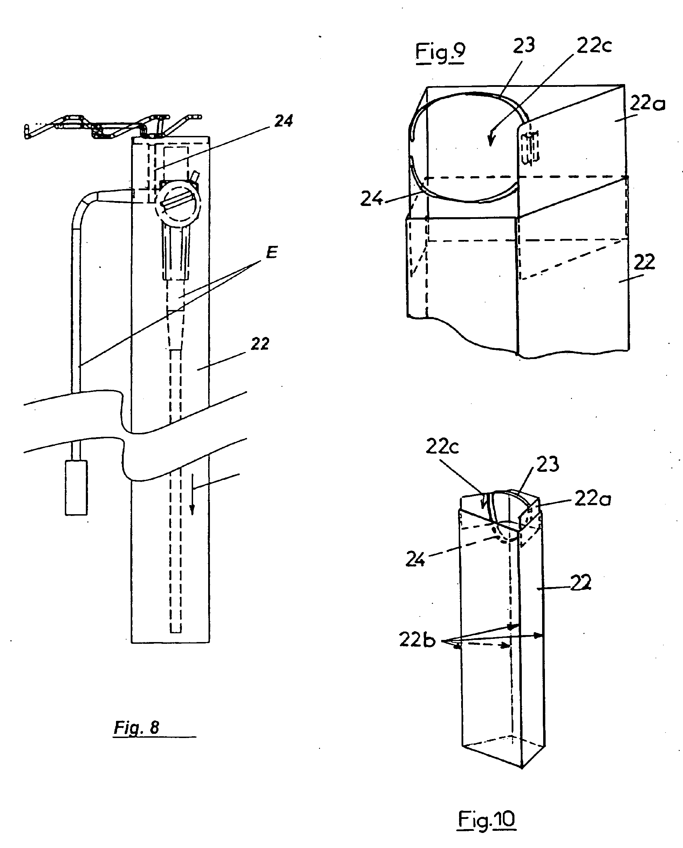

Le compartiment technique 2 est encore équipé :
- en partie basse, d'une prise d'air extérieur 9, avec registre d'admission et d'une sortie d'air 31 (figure 9) permettant l'évacuation de l'excédent d'air ;
- également en partie basse, d'un dispositif de connexion 10 permettant le raccordement de l'armoire à une source d'air médical et d'un dispositif de régulation d'air médical 11 comprenant un régulateur de pression et une électrovanne temporisée ;
- en partie haute, d'un automate 12 de gestion du fonctionnement de l'armoire, accessible à l'extérieur de celle-ci (figure 3).

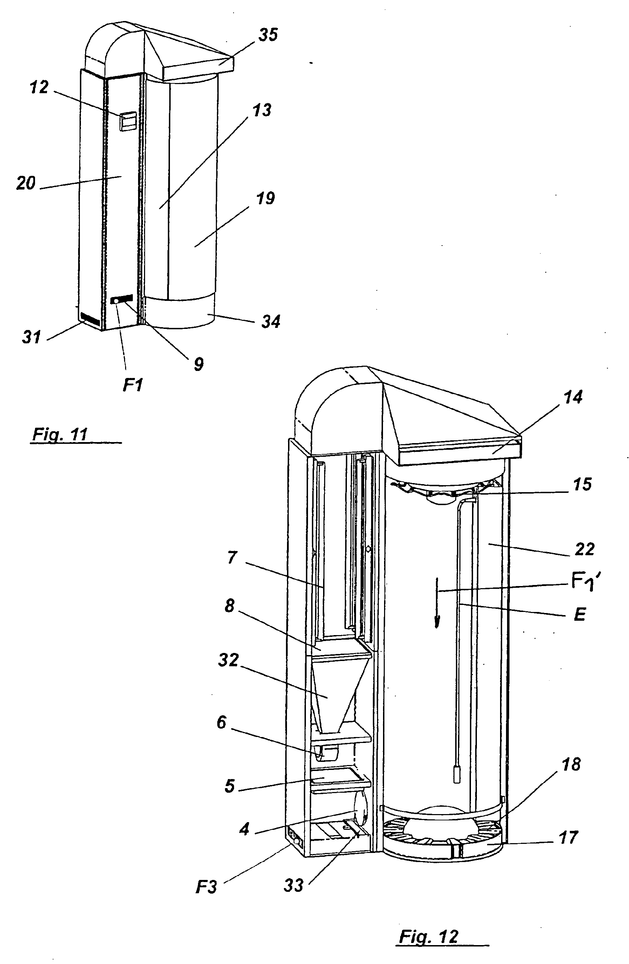

L'enceinte de rangement 3 comprend :
- une ouverture frontale fermée par une porte 13 (figure 11);
- en partie haute : un filtre 14, par exemple de type H14 selon EN 1822, par l'intermédiaire duquel le compartiment technique 2 communique avec l'enceinte de rangement 3, et, dans l'application au rangement des endoscopes E, un système d'accrochage 15 pour la suspension de ces derniers ; un dispositif de répartition d'air médical 16 ;
- en partie basse : un bac amovible 17 de récupération des liquides et une grille de fond 18 disposée au-dessus dudit bac.

La référence 34 (figure 9) désigne un volet d'accès au bac 17. On observe que la grille de fond 18 et le bac 17 sont constitués de deux pièces emboîtées l'une sur l'autre. Le volet 34 permet de retirer l'ensemble, par exemple pour le remplacer par un jeu propre, sans ouvrir totalement l'armoire, ce qui permet de limiter la rétro-contamination.

L'enceinte de rangement est encore avantageusement équipée d'une porte d'accès 19 pour permettre son nettoyage, tandis que le compartiment technique 2 est également équipé d'une porte d'accès 20 pour la maintenance.

La référence 35 désigne un volet d'accès au filtre 14.

On comprend que grâce à la mise en oeuvre de l'armoire qui vient d'être décrite, l'air circule constamment en boucle, grâce au moto-ventilateur 6, depuis le compartiment technique 2 vers l'enceinte de stockage 3 dans laquelle il est distribué sous forme de flux laminaires, du haut vers le bas de ladite enceinte d'où il est réintroduit dans la partie basse du compartiment technique 2. Le registre 9 correctement calibré autorise, notamment grâce à l'action d'aspiration du moto-ventilateur 6, une admission d'air ambiant dans la partie basse du compartiment technique, cet apport d'air neuf se mélange à l'air recyclé, dans ladite partie basse et permet, d'une part, la mise en surpression de l'enceinte de stockage et, d'autre part, la dissipation d'une partie de la chaleur induite par le moto-ventilateur 6 et la batterie 7 de tubes UV. L'air est réintroduit dans le compartiment technique 2 à travers une ouverture ménagée dans la partie inférieure de la cloison séparant ledit compartiment technique et l'enceinte de rangement 3. Cette ouverture est équipée d'une grille obturable 33.

Cette grille obturable 33 est munie d'un volet anti-retour actionné par un électro-aimant relié au moto-ventilateur 6, de sorte que l'arrêt du fonctionnement de ce dernier (par suite de panne ou autre cause) provoque la fermeture de la grille 33 et l'isolation de l'enceinte de rangement 3, ce qui supprime toute possibilité de rétro-contamination.

La vitesse de rotation du motoventilateur 6 est asservie à la mesure de pression différentielle prise en amont et en aval du dispositif de filtration 14, par exemple au moyen d'un pressostat différentiel (non représenté).

Dans l'application au rangement des endoscopes aseptisés, l'air médical fourni par l'établissement hospitalier sert à parfaire le séchage desdits endoscopes. Il est canalisé vers ces derniers, via le dispositif de régulation d'air médical 11 et le répartiteur 16 qui peut être incorporé au système d'accrochage des endoscopes situé dans la partie haute de l'enceinte de stockage. La connexion du répartiteur 16 avec les endoscopes est réalisée au moyen d'un tube de silicone 21 et d'un raccord ou coupleur rapide 36 muni d'un obturateur.

Selon l'invention, l'armoire de stockage hyperaseptique comprend, selon une importante disposition caractéristique, des manches stériles 22, à usage unique, pour protéger individuellement les endoscopes d'un contact accidentel pendant l'entreposage ou le transport.

Ces manches stériles que l'on désigne sous le nom de "saches" dans la suite du présent exposé, sont constituées, chacune, par un tube ou gaine de forme allongée, ouverte à ses extrémités supérieure et inférieure. Ces saches stériles sont dimensionnées pour autoriser le logement d'un endoscope E préalablement désinfecté et plus particulièrement le logement d'au moins la partie invasive E' de ce dernier dont la partie non invasive E" reste à l'extérieur des saches. Elles présentent un diamètre ou section très nettement supérieur au diamètre de la partie invasive E' des endoscopes qu'elles sont appelées à protéger.

Elles peuvent comporter des plis longitudinaux 22b, de sorte à présenter une forme prismatique lorsqu'elles sont suspendues.

La sache selon l'invention est, de préférence, réalisée dans un matériau souple, non poreux, et d'une épaisseur lui permettant de prendre et de conserver une forme tubulaire stable, lorsqu'elle est placée en position verticale, afin de ne pas "coller" à l'endoscope qu'elle abrite. L'extrémité supérieure 22a de la sache est renforcée ou "rigidifiée" par tout procédé et/ou moyen adéquat, afin de la maintenir ouverte de façon permanente. La portion supérieure renforcée 22a peut comporter une ouverture latérale 22c facilitant l'accès à l'intérieur de la sache.

De la sorte, la sache stérile 22 sert à canaliser le flux d'air laminaire amené à l'entrée supérieure de cette dernière, ce flux d'air étant contrôlé d'un point de vue hygiène, par tout procédé convenable connu en soi, de façon à mettre l'intérieur de ladite sache et donc l'environnement proche de la partie invasive E' de l'endoscope, en situation isolée de l'environnement extérieur pouvant être contaminant.

La partie supérieure 22a de la sache est munie d'un système de suspension 23 qui peut être constitué par une bride ou arceau souple permettant de suspendre, en station verticale, l'ensemble formé de ladite sache et de l'endoscope à un système d'accrochage équipant l'armoire précédemment décrite.

D'autre part, la partie supérieure de la sache 22 est munie d'un moyen pour la suspension d'un endoscope E. Ce moyen est, par exemple, constitué par une lanière d'accrochage 24 disposée en travers de l'ouverture de la partie supérieure renforcée 22a de la sache 22, au-dessous de la bride ou arceau 23. Cette disposition permet l'accrochage d'un endoscope E placé en chevauchement sur la lanière 24, par l'intermédiaire de son extrémité fermée correspondant à la zone de jonction de sa sonde ou partie invasive E' et de son câble optique extérieur E".

Compte tenu du fait que des accessoires (connecteurs, écouvillons, etc.) sont associés à chaque endoscope, la partie supérieure 22a de chaque sache 22 peut être munie d'une pochette (non représentée) pouvant recevoir ces accessoires de façon à rester solidaire de chaque endoscope concerné lors de son stockage.

Selon le mode d'exécution illustré aux figures 2, 4, 5, 8, 10 et 12, le système d'accrochage 15 des endoscopes est constitué par un tourniquet dont l'arbre axial vertical 25 est monté tournant dans des paliers installés dans la partie supérieure et, éventuellement, dans la partie inférieure, respectivement, de l'enceinte de rangement ou de chaque enceinte de rangement.

Ce tourniquet peut être entraîné en rotation manuellement ou par l'intermédiaire d'une motorisation appropriée, par exemple au moyen d'un moto-réducteur électrique 30, avec ou sans indexation, afin de limiter les manipulations à l'intérieur de l'enceinte de stockage.

La partie supérieure porteuse du tourniquet 15 peut être constituée par des bras radiaux 26 solidaires de l'arbre axial 25 par l'intermédiaire de l'une de leurs extrémités et sur l'autre extrémité desquels est fixé un support périphérique 27 pour l'accrochage du moyen de suspension 23 des manches ou saches stériles 22.

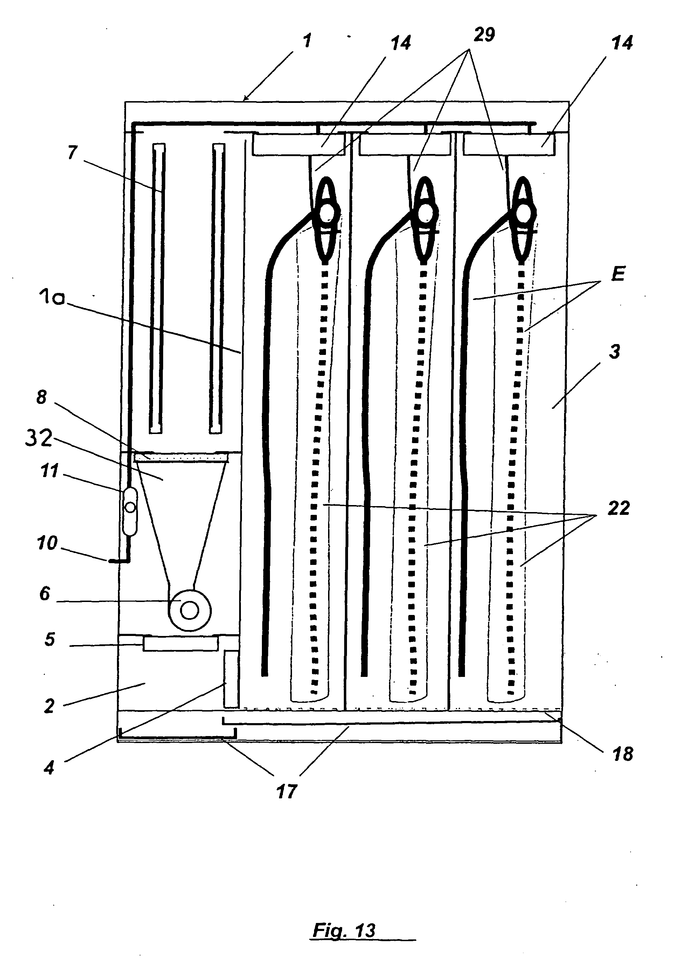

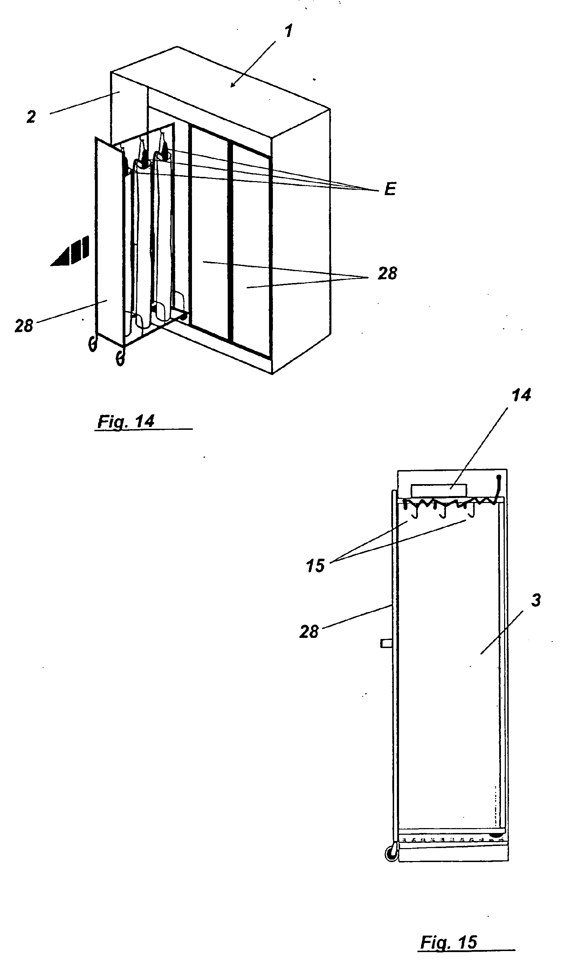

Le mode d'exécution illustré aux figures 13 à 15 diffère de celui qui vient d'être décrit principalement par le fait que les moyens de stockage des endoscopes sont constitués par des tiroirs ou racks 28 (au nombre de trois selon l'exemple représenté) montés avec une aptitude de coulissement dans l'enceinte de stockage 3.

Ces tiroirs sont équipés de moyens de suspension 29, par exemple du genre porte-manteaux, et leur face frontale ou porte constitue, en position de fermeture, une partie de la paroi antérieure délimitant l'enceinte de rangement 3.

L'armoire de stockage aseptique selon l'invention peut encore être équipée de l'un et/ou l'autre des dispositifs ci-après :
- une sonde de température avec seuil de coupure sur alarme haute, installée dans l'enceinte de stockage hyperaseptique et permettant de monitorer la température à l'intérieur de celle-ci ;
- une sonde d'humidité disposée à l'intérieur de l'enceinte de stockage hyperaseptique, permettant de contrôler l'hygrométrie à l'intérieur de cette dernière, afin de prévoir la régénération de la capsule dessicative.

II est aussi possible de relier l'armoire hyperaseptique selon l'invention à un système de traçabilité (enregistrement de données), connu en soi, permettant l'enregistrement des opérations d'ouverture/fermeture de l'armoire.

## Revendications

1. Armoire de stockage hyperaseptique, en particulier pour endoscopes, du genre comprenant un compartiment technique (2) contenant les composants techniques (4, 5, 7, 8) nécessaires à la production d'air aseptisé, une enceinte de rangement (3) pour la réception et le stockage des articles (E), en particulier des endscopes, à préserver de toute contamination, cette enceinte communiquant, en partie haute et en partie basse, avec ledit compartiment technique (2), et des moyens (6) permettant d'obtenir une circulation en boucle de l'air à travers ledit compartiment et à travers ladite enceinte, jusqu'en partie basse de cette dernière, d'où il repasse dans la partie inférieure dudit compartiment technique (2) dans lequel il circule de bas en haut à travers lesdits composants techniques de traitements (4,5,7,8), ladite armoire (1) étant agencée de sorte que l'air traité soit introduit à la partie supérieure de l'enceinte de stockage et poussé à circuler de haut en bas, sous forme de flux laminaire et en surpression à travers ladite enceinte de stockage (3) , **caractérisée en ce que** ladite armoire comprend encore des manches verticales stériles (22), à usage unique, disposées verticalement dans l'enceinte de rangement (3), ces manches stériles, aussi appelées saches, étant constituées par des gaines de forme allongée ouvertes à leurs extrémités supérieure et inférieure, et dimensionnées pour autoriser en particulier le logement d'au moins la partie invasive (E') d'un endoscope (E) désinfecté, l'extrémité supérieure (22a) de ces saches étant munie d'un moyen permettant leur suspension (23) à un support (26-27) installé en partie haute de ladite enceinte de rangement, lesdites saches permettant ainsi de canaliser l'écoulement laminaire du flux d'air aseptisé autour des endoscopes logés dans ces dernières.

2. Armoire de stockage hyperaseptique selon la revendication 1, **caractérisée en ce que** les saches stériles (22) sont constituées par des gaines exécutées dans un matériau souple, de préférence non poreux, et d'une épaisseur leur permettant de prendre et de conserver une forme tubulaire stable lorsqu'elles sont placées en position verticale.

3. Armoire de stockage hyperaseptique, suivant l'une des revendications 1 ou 2, **caractérisée en ce qu'**elle est munie d'un dispositif de connexion (10) permettant son raccordement à une source d'air médical et **en ce qu'**un dispositif de répartition d'air médical (16) est installé dans l'enceinte de rangement (3), de façon à permettre son raccordement aux appareils (E) stockés dans ladite enceinte, en attente de réutilisation.

4. Armoire de stockage hyperaseptique selon la revendication 1, suivant laquelle le compartiment technique (2) est muni, dans sa partie basse, d'un registre (9) d'admission d'air ambiant, **caractérisée en ce que** ledit compartiment technique (2) est, également, pourvu, d'une ouverture de sortie (31) de l'excédent d'air, disposée dans sa partie basse.

5. Armoire de stockage hyperaseptique, selon l'une des revendications 1 ou 4, **caractérisée en ce que** l'ouverture inférieure établissant une communication entre l'enceinte de rangement (3) et le compartiment technique (2) est munie d'une grille obturable (33) dont le volet obturateur est asservi à un dispositif de commande assurant sa fermeture automatique lors de l'arrêt du fonctionnement des moyens (6) assurant la circulation de l'air en boucle.

6. Armoire de stockage hyperaseptique suivant l'une quelconque des revendications 1, 4 ou 5, **caractérisée en ce que** les composants techniques (5, 7, 8) installés dans le compartiment technique (2) comprennent, notamment, de bas en haut :
- un filtre de pré-filtration (5), par exemple de type gravimétrique G3 selon EN 779 ;
- un filtre (8) haute efficacité par exemple de type G4 selon EN 779 ;
- une batterie de tubes UV (7) pour le traitement de l'air ;
- et, de préférence, un divergeant (32) disposé au-dessous dudit filtre haute-efficacité (8).

7. Armoire de stockage hyperaseptique selon la revendication 6, **caractérisée en ce que** lesdits composants techniques comprennent également un dispositif de déshydratation (4) disposé au-dessous du filtre de pré-filtration (5), ce dispositif de déshydratation étant, par exemple, constitué par un dessiccateur chimique, ou par une batterie froide de déshumidification.

8. Armoire de stockage hyperaseptique selon l'une quelconque des revendications 1 ou 4 à 7, **caractérisée en ce que** les composants techniques nécessaires à la production d'air aseptisé comprennent encore un filtre très haute efficacité (14), par exemple de type H14 selon EN 1822, disposé dans la partie supérieure de l'enceinte de rangement (3) et par l'intermédiaire duquel le compartiment technique (2) communique avec ladite enceinte.

## Claims

1. A hyperaseptic storage cabinet, in particular for endoscopes, of the kind comprising a technical compartment (2) containing the technical components (4, 5, 7, 8) necessary for the production of asepticised air, an arrangement enclosure (3) for receiving and storing the articles (E), in particular endoscopes, to be preserved from any contamination, said enclosure communicating in the upper part and in the lower part with said technical compartment (2), and means (6) for producing a loop circulation of the air through said compartment and through said enclosure into the lower part of the latter from which it passes again into the lower part of said technical compartment (2) in which it circulates upwardly through said technical treatment components (4, 5, 7, 8), said cabinet (1) being arranged in such a way that the treated air is introduced in the upper part of the storage enclosure and urged to circulate in a downward direction in the form of a laminar flow and under an increased pressure through said storage enclosure (3), **characterised in that** said cabinet also comprises vertical single-use sterile sleeves (22) disposed vertically in the arrangement enclosure (3), said sterile sleeves, also referred to as packs, being formed by sheaths of elongate shape which are open at their upper and lower ends and dimensioned to permit in particular the accommodation therein of at least the invasive part (E') of a disinfected endoscope (E), the upper end (22a) of said packs being provided with a means permitting suspension thereof (23) from a support (26-27) installed in the upper part of said arrangement enclosure, said packs thus permitting channelling of the laminar stream of the asepticised air flow around the endoscopes accommodated in the packs.

2. A hyperaseptic storage cabinet according to claim 1 **characterised in that** the sterile packs (22) are formed by sheaths made of a flexible material which is preferably non-porous, and of a thickness permitting them to assume and retain a stable tubular shape when they are placed in a vertical position.

3. A hyperaseptic storage cabinet according to one of claims 1 and 2 **characterised in that** it is provided with a connecting device (10) permitting connection thereof to a source of medical air and that a medical air distribution device (16) is installed in the arrangement enclosure (3) in such a way as to permit connection thereof to the apparatuses (E) stored in said enclosure in a condition of awaiting re-use.

4. A hyperaseptic storage cabinet according to claim 1 wherein the technical compartment (2) is provided in its lower part with an ambient air intake control (9) **characterised in that** said technical compartment (2) is also provided with an outlet opening (31) for the excess of air, which is disposed in its lower part.

5. A hyperaseptic storage cabinet according to one of claims 1 and 4 **characterised in that** the lower opening establishing a communication between the arrangement enclosure (3) and the technical compartment (2) is provided with a closable grill (33), the closure flap of which is under the control of an actuating device providing for automatic closure thereof when operation of the means (6) providing for the loop air circulation stops.

6. A hyperaseptic storage cabinet according to any one of claims 1, 4 and 5 **characterised in that** the technical components (5, 7, 8) installed in the technical compartment (2) comprise, in particular in an upward direction:
- a pre-filtration filter (5), for example of gravimetric type G3 in accordance with EN 779;
- a high-efficiency filter (8), for example of type G4 in accordance with EN 779;
- an array of UV tubes (7) for treatment of the air;
- and preferably a divergent diffuser means (32) disposed below said high-efficiency filter (8).

7. A hyperaseptic storage cabinet according to claim 6 **characterised in that** said technical components also comprise a dehydration device (4) disposed below the pre-filtration filter (5), said dehydration device being for example formed by a chemical desiccator or by a cold dehumidification array.

8. A hyperaseptic storage cabinet according to any one of claims 1 or 4 to 7 **characterised in that** the technical components necessary for the production of asepticised air further comprise a very high-efficiency filter (14), for example of type H14 in accordance with EN 1822, which is disposed in the upper part of the arrangement enclosure (3) and by way of which the technical compartment (2) communicates with said enclosure.

## Patentansprüche

1. Schrank zur hyperaseptischen Lagerung, insbesondere von Endoskopen, der Art, die ein Technikfach (2), das die technischen Komponenten (4, 5, 7,8) enthält, die für die Erzeugung von aseptischer Luft notwendig sind, einen Schrankraum (3) für die Aufnahme und die Lagerung der Artikel (E), insbesondere von Endoskopen, die vor jeder Kontaminierung zu schützen sind, wobei dieser Raum im oberen Teil und im unteren Teil mit dem Technikfach (2) in Verbindung steht, und Mittel (6) umfasst, die es ermöglichen, einen Zirkulationskreislauf der Luft durch das Fach und den Raum bis zum unteren Teil des Letzteren zu erhalten, von wo aus er wieder in den unteren Teil des Technikfachs (2) zurückkehrt, in dem er von unten nach oben durch die technischen Aufbereitungskomponenten (4, 5, 7, 8) zirkuliert, wobei der Schrank (1) so angeordnet ist, dass die aufbereitete Luft am oberen Teil des Lagerraums eingeführt und dazu gedrängt wird, von oben nach unten in Form einer laminaren und unter Überdruck stehenden Strömung durch den Lagerraum (3) zu zirkulieren, **dadurch gekennzeichnet, dass** der Schrank außerdem vertikale sterile Hülsen (22) zum einmaligen Gebrauch umfasst, die vertikal in dem Schrankraum (3) angeordnet sind, wobei diese sterilen Hülsen, die auch als "Beutel" bezeichnet werden, aus Hüllen mit länglicher Form gebildet sind, die an ihrem oberen und unteren Ende offen und so dimensioniert sind, dass sie insbesondere die Aufnahme zumindest des invasiven Teils (E') eines desinfizierten Endoskops (E) gestatten, wobei das obere Ende (22a) dieser Beutel mit einem Mittel ausgestattet ist, das ihr Aufhängen (23) an einer Halterung (26 - 27) gestattet, die im oberen Teil des Schrankraums so installiert ist, dass die Beutel das Kanalisieren des laminaren Abfließens des aseptischen Luftstroms um die in den Letzteren befindlichen Endoskope gestatten.

2. Schrank zur hyperaseptischen Lagerung nach Anspruch 1**, dadurch gekennzeichnet, dass** die sterilen Beutel (22) aus Hüllen gebildet werden, die aus einem weichen Material ausgeführt sind, das vorzugsweise nicht porös ist und eine Dicke aufweist, die es ihnen gestattet, dass sie eine stabile röhrenförmige Form annehmen und beibehalten können, wenn sie in vertikale Position gebracht werden.

3. Schrank zur hyperaseptischen Lagerung nach einem der Ansprüche 1 oder 2, **dadurch gekennzeichnet, dass** er mit einer Verbindungsvorrichtung (10) ausgestattet ist, die seinen Anschluss an eine medizinische Luftquelle gestatten, und dadurch, dass eine Verteilervorrichtung für medizinische Luft (16) in dem Schrankraum (3) so installiert ist, dass ihr Anschluss an Geräte (E) ermöglicht wird, die in dem Raum bis zu ihrer erneuten Verwendung gelagert werden.

4. Schrank zur hyperaseptischen Lagerung nach Anspruch 1, bei welchem das Technikfach (2) in seinem unteren Teil mit einem Einlassschieber (9) für Umgebungsluft ausgestattet ist, **dadurch gekennzeichnet, dass** das Technikfach (2) auch mit einer Auslassöffnung (31) für den Luftüberschuss versehen ist, die in seinem unteren Teil angeordnet ist.

5. Schrank zur hyperaseptischen Lagerung nach einem der Ansprüche 1 oder 4, **dadurch gekennzeichnet, dass** die untere Öffnung, die eine Verbindung zwischen dem Schrankraum (3) und dem Technikfach (2) herstellt, mit einem verschließbaren Gitter (33) ausgestattet ist, dessen Verschlussschieber von einer Steuereinrichtung angesteuert wird, die das automatische Schließen beim Abschalten des Betriebs der Mittel (6) sicherstellt, die den Zirkulationskreislauf der Luft sicherstellen.

6. Schrank zur hyperaseptischen Lagerung nach einem der Ansprüche 1, 4 oder 5, **dadurch gekennzeichnet, dass** die technischen Komponenten (5, 7, 8), die im Technikfach (2) installiert sind, insbesondere von unten nach oben Folgendes umfassen:
- ein Filter zur Vorfilterung (5), beispielsweise des gravimetrischen Typs G3 gemäß EN 779;
- ein hocheffizientes Filter (8), beispielsweise des Typs G4 gemäß EN 779;
- eine Reihe von UV-Röhren (7) zur Aufbereitung der Luft;
- und vorzugsweise eine Divergiereinrichtung (32), die unter dem hocheffizienten Filter (8) angeordnet ist.

7. Schrank zur hyperaseptischen Lagerung nach Anspruch 6, **dadurch gekennzeichnet, dass** die technischen Komponenten des Weiteren eine Dehydriereinrichtung (4) umfassen, die unter dem Filter zur Vorfilterung (5) angeordnet ist, wobei diese Dehydriereinrichtung beispielsweise aus einem chemischen Trockenmittel oder einem Flächenkühler zur Entfeuchtung gebildet wird.

8. Schrank zur hyperaseptischen Lagerung nach einem der Ansprüche 1 oder 4 bis 7, **dadurch gekennzeichnet, dass** die technischen Komponenten, die für die Erzeugung von aseptischer Luft notwendig sind, außerdem ein Filter von sehr hoher Wirksamkeit (14), beispielsweise des Typs H14 gemäß EN 1822, umfassen, das im oberen Teil des Schrankraums (3) angeordnet ist, über welches das Technikfach (2) mit dem Raum in Verbindung steht.
